(19) Europäisches Patentamt
European Patent Office
Office européen des brevets

(11) Veröffentlichungsnummer: **0 393 445 A2**

(12) # EUROPÄISCHE PATENTANMELDUNG

(21) Anmeldenummer: **90106679.5**

(22) Anmeldetag: **06.04.90**

(51) Int. Cl.5: **A61K 37/64**

(30) Priorität: **19.04.89 DE 3912829**

(43) Veröffentlichungstag der Anmeldung:
**24.10.90 Patentblatt 90/43**

(84) Benannte Vertragsstaaten:
**AT BE CH DE DK ES FR GB GR IT LI LU NL SE**

(71) Anmelder: **BAYER AG**
**D-5090 Leverkusen 1 Bayerwerk(DE)**

(72) Erfinder: **Benz, Günter, Dr.**
**Am Bolkumer Busch 15**
**D-5620 Velbert 15(DE)**
Erfinder: **Bender, Wolfgang, Dr.**
**Claudiusweg 5**
**D-5600 Wuppertal 1(DE)**
Erfinder: **Henning, Rolf, Dr.**
**Böcklinstrasse 16**
**D-5600 Wuppertal 1(DE)**
Erfinder: **Paessens, Arnold, Dr.**
**Stresemannstrasse 51**
**D-5657 Haan(DE)**

(54) **Verwendung von renininhibitorischen Peptiden als Mittel gegen Retroviren.**

(57) Die vorliegende Erfindung betrifft die Verwendung von bekannten renininhibitorischen Peptiden als antivirale Mittel in der Human- und Tiermedizin.

EP 0 393 445 A2

# Verwendung von renininhibitorischen Peptiden als Mittel gegen Retroviren

Die vorliegende Erfindung betrifft die Verwendung von bekannten renininhibitorischen Peptiden als antivirale Mittel in der Human- und Tiermedizin.

Es ist bereits bekannt, daß die im folgenden aufgeführten renininhibitorischen Peptide eine kreislaufbeeinflussende Wirkung besitzen und zur Behandlung des Bluthochdrucks und der Herzinsuffizienz eingesetzt werden können [vgl. EP 01 04 041; EP 01 44 290; EP 00 77 029; EP 00 81 783; EP 01 11 266; EP 00 77 028; EP-A2 01 72 347; EP-A2 01 81 071; EP-A2 02 11 586; EP-A2 02 23 437; EP-A2 01 32 304; EP-A1 01 18 223].

Es wurde nun gefunden, daß Peptide der allgemeinen Formel (I)

X-A-B-D-Y-E-F-Z (I)

in welcher

X - für Wasserstoff, geradkettiges oder verzweigtes Alkyl, Alkylsulfonyl oder Alkoxycarbonyl mit bis zu 10 Kohlenstoffatomen steht,

- für eine Gruppe der Formel $-COR^1$ steht,

worin

$R^1$ - geradkettiges oder verzweigtes Alkyl mit bis zu 8 Kohlenstoffatomen bedeutet, das gegebenenfalls durch Hydroxy, Amino oder Aryl mit 6 bis 10 Kohlenstoffatomen substituiert ist,

- für Phenylsulfonyl, Tolylsulfonyl oder Naphthyl steht, oder

- für eine Aminoschutzgruppe steht;

A - für eine direkte Bindung steht, oder

- für einen Rest der Formel

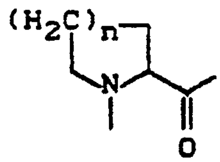 steht,

worin

n eine Zahl 1 oder 2 bedeutet,

- für eine Gruppe der Formel

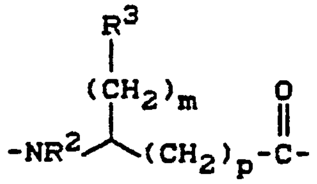 steht,

worin

m eine Zahl 0, 1, 2, 3 oder 4 bedeutet,

p eine Zahl 0 oder 1 bedeutet,

$R^2$ - Wasserstoff, geradkettiges oder verzweigtes Alkyl mit bis zu 8 Kohlenstoffatomen, Naphthyl oder eine Aminoschutzgruppe bedeutet,

$R^3$ - Wasserstoff, Naphthyl, Halogen, Carboxy, geradkettiges oder verzweigtes Alkyl oder Alkylthio mit bis zu 8 Kohlenstoffatomen bedeutet, das gegebenenfalls durch Mercapto, Alkylcarbonyl mit bis zu 8 Kohlenstoffatomen, Hydroxy oder Halogen substituiert ist, oder

- eine Gruppe der Formel $-CH_2-NHR^4$ bedeutet,

worin

$R^4$ - Wasserstoff, geradkettiges oder verzweigtes Alkyl oder Alkylsulfonyl mit bis zu 8 Kohlenstofftomen, Phnylsulfonyl oder eine Aminoschutzgruppe bedeutet,

- Guanidino, Indolyl, Imidazolyl, Pyridyl, Triazolyl oder Pyrazolyl bedeutet, die gegebenenfalls durch $R^4$ substituiert sind,

wobei

$R^4$ die oben angegebene Bedeutung hat,

- Aryl mit 6 bis 10 Kohlenstoffatomen bedeutet, das gegebenenfalls bis zu 3-fach gleich oder verschieden durch geradkettiges oder verzweigtes Alkyl oder Alkoxy mit bis zu 8 Kohlenstoffatomen, Halogen, Hydroxy, Nitro oder durch eine Gruppe der Formel $-NR^5R^6$ substituiert ist,

worin

$R^5$ und $R^6$ gleich oder verschieden sind und Wasserstoff oder geradkettiges oder verzweigtes Alkyl mit bis zu 8 Kohlenstoffatomen bedeuten,

B, E und F gleich oder verschieden sind und die oben angegebene Bedeutung von A haben und mit dieser gleich oder verschieden sind, in ihrer D-Form, L-Form oder D,L-Isomerengemisch, bevorzugt in der L-Form und

D - die oben angegebene Bedeutung von A hat und mit dieser gleich oder verschieden ist, in seiner D-Form, L-Form oder als D,L-Isomerengemisch, mit der Maßgabe, daß D nicht für eine direkte Bindung und nicht für Asparagin (Asn) stehen darf,

Y - für eine Gruppe der Formel

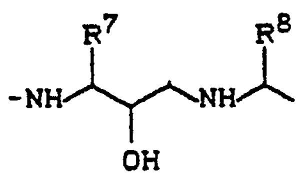 , 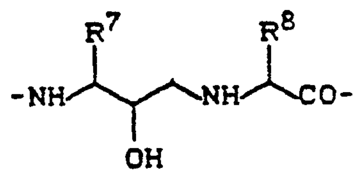

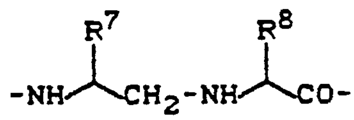 , 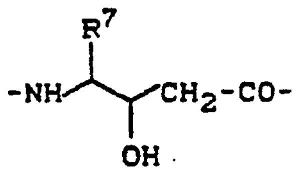

oder 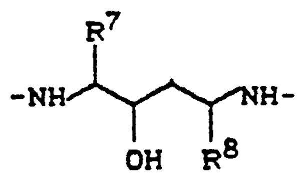 steht,

worin

$R^7$ - geradkettiges oder verzweigtes Alkyl mit bis zu 12 Kohlenstoffatomen bedeutet, das gegebenenfalls durch Cycloalkyl mit 3 bis 8 Kohlenstoffatomen Aryl mit 6 bis 10 Kohlenstoffatomen, Halogen, Hydroxy, Nitro oder durch einen gesättigten oder ungesättigten 5-bis 7-gliedrigen Heterocyclus mit bis zu 4 Heteroatomen aus der Reihe Sauerstoff, Schwefel oder Stickstoff substituiert ist,

- Aryl mit 6 bis 10 Kohlenstoffatomen bedeutet, das gegebenenfalls durch Halogen, Nitro, Hydroxy, Cyano, geradkettiges oder verzweigtes Alkyl oder Alkoxy mit bis zu 8 Kohlenstoffatomen oder durch eine Gruppe der Formel $-NR^5R^6$ substituiert ist,

worin

$R^5$ und $R^6$ die oben angegebene Bedeutung haben,

$R^8$ - Wasserstoff, geradkettiges oder verzweigtes Alkyl mit bis zu 8 Kohlenstoffatomen bedeutet, das gegebenenfalls durch Hydroxy, Cycloalkyl mit 3 bis 8 Kohlenstoffatomen und/oder Aryl mit 6 bis 10 Kohlenstoffatomen substituiert ist, welches seinerseits durch Nitro, Cyano oder Hydroxy substituiert ist,

Z - für eine Aminoschutzgruppe steht,

- für geradkettiges oder verzweigtes Alkyl oder Alkoxy mit bis zu 10 Kohlenstoffatomen steht, die gegebenenfalls durch Hydroxy, Phenyl oder Pyridyl substituiert sind,

- für eine Gruppe der Formel $-NR^9R^{10}$ oder $-COR^{11}$ steht,

worin

$R^9$ und $R^{10}$ gleich oder verschieden sind und Wasserstoff, geradkettiges oder verzweigtes Alkyl oder Alkenyl mit bis zu 10 Kohlenstoffatomen bedeuten, die gegebenenfalls durch Pyridyl oder Hydroxy substituiert sind, oder einen gesättigten 5-bis 7-gliedrigen Heterocyclus mit bis zu 3 Heteroatomen aus der Reihe Schwefel, Sauerstoff oder Stickstoff bedeuten, der gegebenenfalls durch Benzyl oder Pyridyl substituiert ist,

$R^{11}$ geradkettiges oder verzweigtes Alkyl mit bis zu 8 Kohlenstoffatomen bedeutet, das gegebenenfalls durch Pyridyl oder durch eine Gruppe der Formel $-NR^9R^{10}$ substituiert ist,
worin
$R^9$ und $R^{10}$ die oben angegebene Bedeutung haben,
- für Adamantyl oder für einen gesättigten oder ungesättigten 7-gliedrigen Heterocyclus mit bis zu 3 Heteroatomen aus der Reihe Sauerstoff, Schwefel oder Stickstoff steht, der gegebenenfalls durch Benzyl substituiert ist,
neben der bekannten renininhibitorischen Aktivität überraschenderweise auch eine gute Wirkung gegen Retroviren haben.

Aminoschutzgruppe steht im Rahmen der Erfindung für die üblichen in der Peptid-Chemie verwendeten Aminoschutzgruppen.

Hierzu gehören bevorzugt: Benzyloxycarbonyl, 4-Brombenzyloxycarbonyl, 2-Chlorbenzyloxycarbonyl, 3-Chlorbenzyloxycarbonyl, Dichlorbenzyloxycarbonyl,3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-(Di-n-butyl-methylsilyl)-ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Ethylthiocarbonyl, Methylthiocarbonyl, Butylthiocarbonyl, Tert.-Butylthiocarbonyl, Phenylthiocarbonyl, Benzylthiocarbonyl, Methylaminocarbonyl, Ethylaminocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl, Phthaloyl oder Phthalimido.

Besonders bevorzugte Aminoschutzgruppen sind Benzyloxycarbonyl, 3,4-Dimethoxybenzyloxycarbonyl, 3,5-Dimethoxybenzyloxycarbonyl, 4-Methoxybenzyloxycarbonyl, 4-Nitrobenzyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, Hexoxycarbonyl, Octoxycarbonyl, 2-Bromethoxycarbonyl, 2-Chlorethoxycarbonyl, Phenoxyacetyl, Naphthylcarbonyl, Adamatylcarbonyl, Phthaloyl, 2,2,2-Trichlorethoxycarbonyl, 2,2,2-Trichlor-tert-butoxycarbonyl, Menthyloxycarbonyl, Vinyloxycarbonyl, Allyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido oder Isovaleroyl.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate. Bevorzugt liegen die Grupppen B, D, E und F unabhängig voneinander in der optisch reinen, bevorzugt in der L-Form vor.

Die erfindungsgemäßen Verbindungen der allgemeinen Formel (I) können in Form ihrer Salze vorliegen. Dies können Salze der erfindungsgemäßen Verbindungen mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure.

Bevorzugt werden Verbindungen der allgemeinen Formel (I)
in welcher
X - für Wasserstoff, geradkettiges oder verzweigtes Alkyl oder Alkoxycarbonyl mit bis zu 10 Kohlenstoffatomen steht,
- für Naphthyl oder eine Aminoschutzgruppe steht,

A - für eine direkte Bindung steht, oder
- für einen Rest der Formel

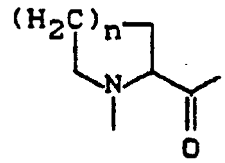 steht,

worin
n eine Zahl 1 oder 2 bedeutet,
- für einen Rest der Formel

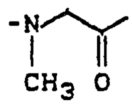 , 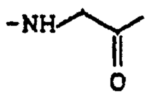 , 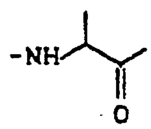 , 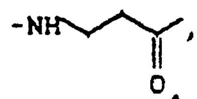 , 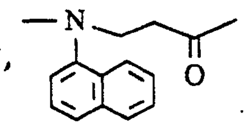

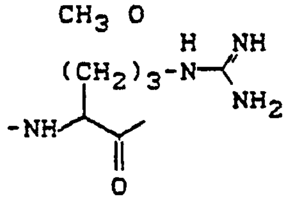 , 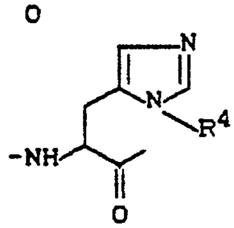 , 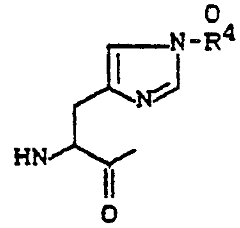

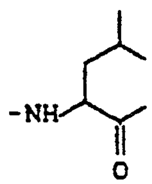 , 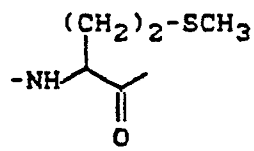 , 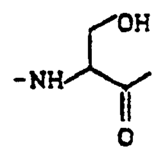

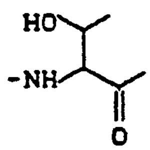 , 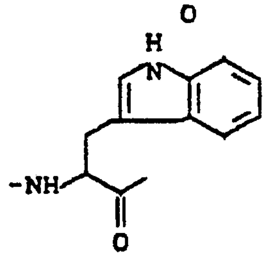 , 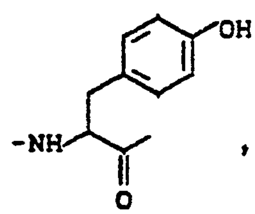 ,

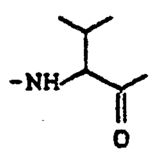 , 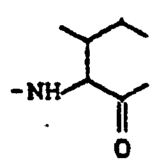 , 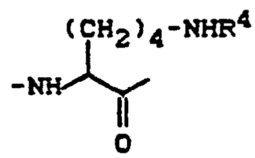

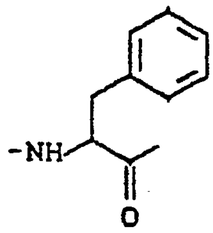 , 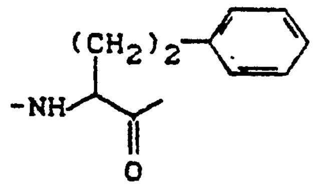 , 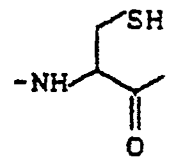 ,

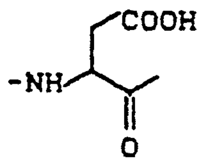 , 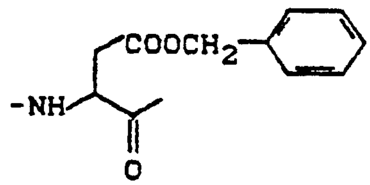 , 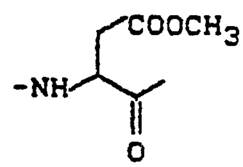 ,

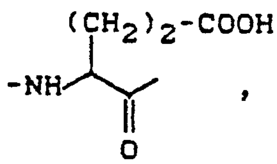 , 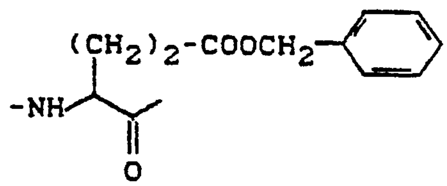 , 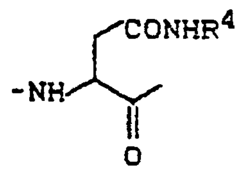 ,

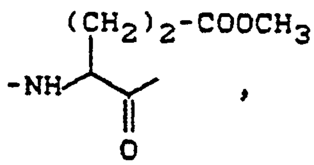 , 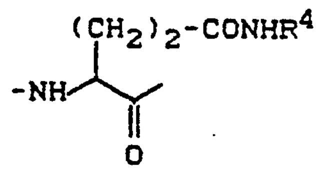 ,

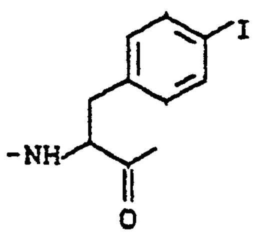 , 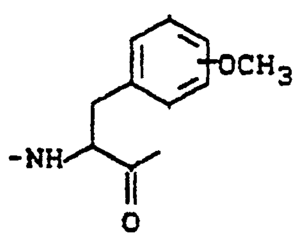 ,

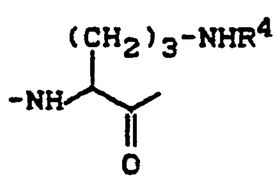 , 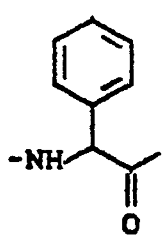 , 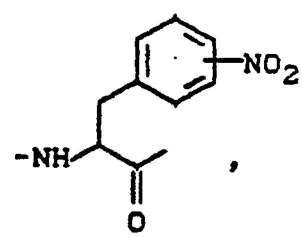 ,

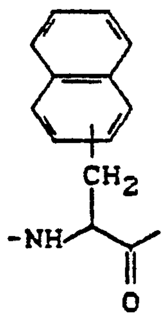 , 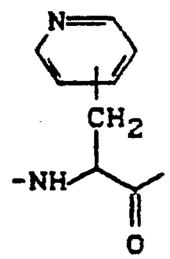 , 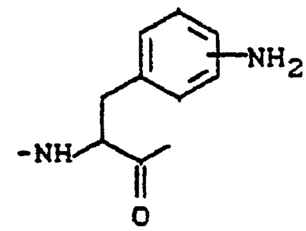

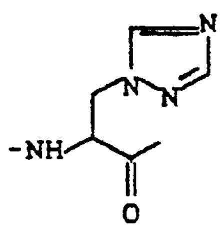 oder 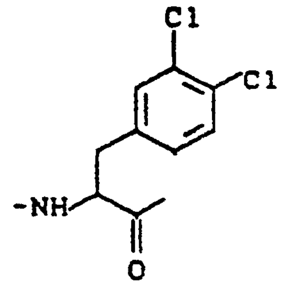

worin

$R^4$ - Wasserstoff, geradkettiges oder verzweigtes Alkyl mit bis zu 6 Kohlenstoffatomen oder eine Aminoschutzgruppe bedeutet,

B, E und F gleich oder verschieden sind und die oben angegebene Bedeutung von A haben und mit dieser gleich oder verschieden sind in ihrer D-Form, L-Form oder als D,L-Isomerengemisch, bevorzugt in der L-Form,

und

D - die oben angegebene Bedeutung von A hat und mit dieser gleich oder verschieden ist, in seiner D-Form, L-Form oder als D,L-Isomerengemisch mit der Maßgabe, daß D nicht für eine direkte Bindung und nicht für Asparagin (Asn) stehen darf,

Y - für eine Gruppe der Formel

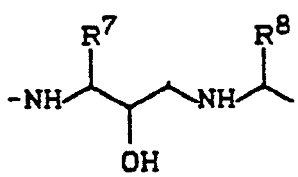 , 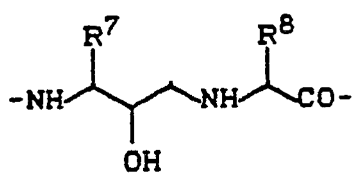

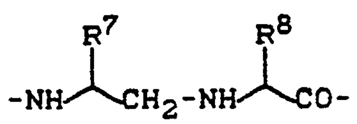 , 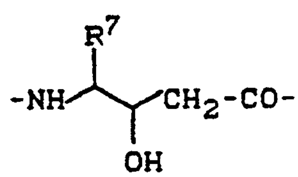

oder 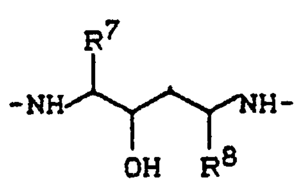 steht,

worin

$R^7$ - geradkettiges oder verzweigtes Alkyl mit bis zu 10 Kohlenstoffatomen bedeutet, das gegebenenfalls durch Cyclopropyl, Cyclopentyl, Cyclohexyl, Phenyl, Hydroxy oder Pyridyl substituiert ist,

$R^8$ - Wasserstoff, geradkettiges oder verweigtes Alkyl mit bis zu 6 Kohlenstoffatomen bedeutet, das gegebenenfalls durch Hydroxy und/oder Phenyl substituiert ist, welches seinerseits durch Nitro, Cyano oder Hydroxy substituiert ist,

Z - für eine Aminoschutzgruppe steht,

- für geradkettiges oder verzweigtes Alkyl oder Alkoxy mit bis zu 8 Kohlenstoffatomen steht, die gegebenenfalls durch Hydroxy, Phenyl oder Pyridyl substituiert sind,
- für eine Gruppe der Formel $-NR^9R^{10}$ oder $-COR^{11}$ steht,
worin
$R^9$ und $R^{10}$ gleich oder verschieden sind und Wasserstoff oder geradkettiges oder verzweigtes Alkyl oder Alkenyl mit bis zu 8 Kohlenstoffatomen bedeuten, die gegebenenfalls durch Pyridyl oder Hydroxy substituiert sind, oder Piperidinyl, Piperazinyl oder Morpholinyl bedeuten, die gegebenenfalls durch Benzyl oder Pyridyl substituiert sind,
$R^{11}$ geradkettiges oder verzweigtes Alkyl mit bis zu 6 Kohlenstoffatomen bedeutet, das gegebenenfalls durch Pyridyl oder durch eine Gruppe der Formel $-NR^9R^{10}$ substituiert ist,
worin
$R^9$ und $R^{10}$ die oben angegebene Bedeutung haben,
- für Adamantyl oder für Piperidinyl, Piperazinyl oder Morpholinyl steht, das gegebenenfalls durch Benzyl substituiert ist
zur Verwendung als antivirale Mittel.

Besonders bevorzugt sind Verbindungen der allgemeinen Formel (I)
in welcher
X - für Wasserstoff oder geradkettiges oder verzweigtes Alkoxycarbonyl mit bis zu 8 Kohlenstoffatomen steht,
- für Naphthyl oder eine Aminoschutzgruppe steht,
A - für eine direkte Bindung steht, oder
- für einen Rest der Formel

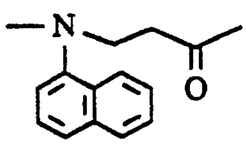

steht
- für Prolyl (Pro), Glycyl (Gly), Alanyl (Ala), β-Alanyl (β-Ala), Arginyl (Arg), Histidyl (His), Asparagin (Asn), Leucyl (Leu), Isoleucyl (Ile), Seryl (Ser), Threonyl (Thr), Tryptophyl (Trp), Tyrosyl (Tyr), Valyl (Val), Lysyl (Lys), Asparagyl (Asp), Glutamyl (Glu), Glutaminamido (Gln), Cystyl (Cys), Methionyl (Met), Phenylalanyl (Phe), 2-, 3-oder 4-Nitrophenylalanyl, 2-, 3- oder 4-Aminophenylalanyl oder Pyridylalanyl steht, die gegebenenfalls durch den Rest $R^4$ substituiert sind,
worin
$R^4$ - Wasserstoff, geradkettiges oder verzweigtes Alkyl mit bis zu 4 Kohlenstoffatomen oder eine Aminoschutzgruppe bedeutet,
B, E und F gleich oder verschieden sind und die oben angegebene Bedeutung von A haben und mit dieser gleich oder verschieden sind in ihrer D-Form,
L-Form oder als D,L-Isomerengemisch, bevorzugt in der L-Form,
und
D - die oben angegebene Bedeutung von A hat und mit dieser gleich oder verschieden ist, in seiner D-Form, L-Form oder als D,L-Isomerengemisch, mit der Maßgabe, daß D nicht für eine direkte Bindung und nicht für Asparagin (Asn) stehen darf,
Y - für eine Gruppe der Formel

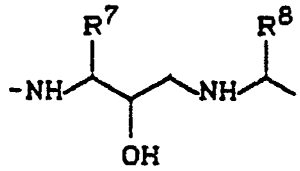 , 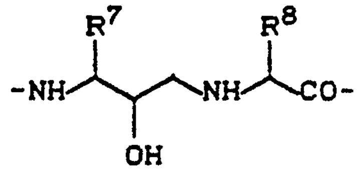

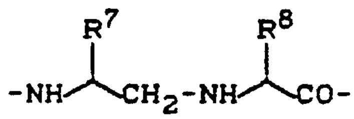 , 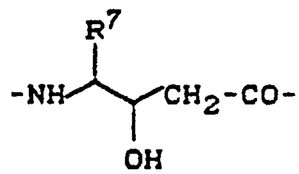

oder 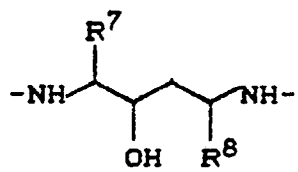 steht,

worin

$R^7$ - geradkettigs odr verzweigtes Alkyl mit bis zu 6 Kohlenstoffatomen bedeutet, das gegebenenfalls durch Cyclohexyl oder Phenyl substituiert ist,

$R^8$ - Wasserstoff, geradkettiges oder verzweigtes Alkyl mit bis zu 4 Kohlenstoffatomen bedeutet, das gegebenenfalls durch Hydroxy und/oder Phenyl substituiert ist, das seinerseits durch Nitro substituiert ist,

Z - für eine Aminoschutzgruppe steht,

- für geradkettiges oder verzweigtes Alkyl oder Alkoxy mit bis zu 6 Kohlenstoffatomen steht, die gegebenenfalls durch Hydroxy, Phenyl oder Pyridyl substituiert sind,

- für eine Gruppe der Formel $-NR^9R^{10}$ oder $-COR^{11}$ steht,

worin

$R^9$ und $R^{10}$ gleich oder verschieden sind und Wasserstoff oder geradkettiges oder verzweigtes Alkyl oder Alkenyl mit bis zu 6 Kohlenstoffatomen bedeuten, die gegebenenfalls durch Pyridyl oder Hydroxy substituiert sind, oder Piperidinyl oder Piperazinyl bedeuten, die gegebenenfalls durch Benzyl substituiert sind,

$R^{11}$ geradkettiges oder verzweigtes Alkyl mit bis zu 4 Kohlenstoffatomen bedeutet, das gegebenenfalls durch Pyridyl oder durch eine Gruppe der Formel $-NR^9R^{10}$ substituiert ist,

worin

$R^9$ und $R^{10}$ die oben angegebene Bedeutung haben,

- für Adamantyl oder für Piperidinyl oder Piperazinyl steht, das gegebenenfalls durch Benzyl substituiert ist,

zur Verwendung als antivirale Mittel.

Ganz besonders bevorzugt für die Verwendung als antivirale Mittel sind die in der Tabelle 1 aufgeführten Peptide:

| Bsp.-Nr. | X | A | B | D | Y | E | F | Z |
|---|---|---|---|---|---|---|---|---|
| 1 | Boc | Pro | Phe | His | 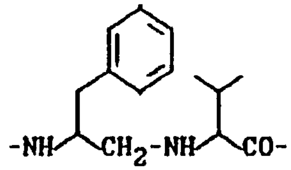 | Ile | - | 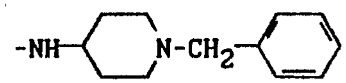 |
| 2 | Boc | Pro | Phe | His | 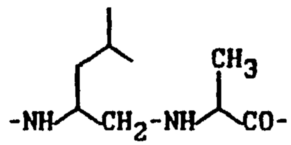 | Ile | - | 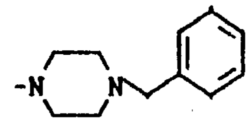 |
| 3 | Boc | - | Boc-His | Boc<br>-His | 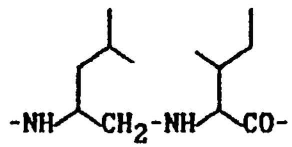 | Ile | - | 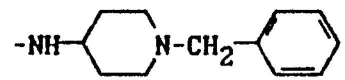 |
| 4 | H | - | - | His | 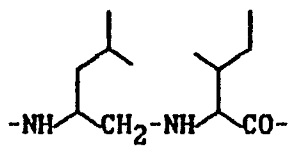 | Ile | - | 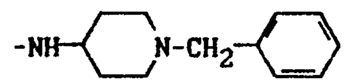 |
| 5 | Boc | - | Phe | His | 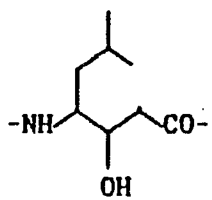 | Leu | - | $-OCH_3$ |

| Bsp.-Nr. | X | A | B | D | Y | E | F | Z |
|---|---|---|---|---|---|---|---|---|
| 6 | Boc | - | - | - | 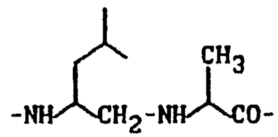 | Ile | - | 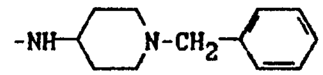 |
| 7 | Boc | - | Phe | His | 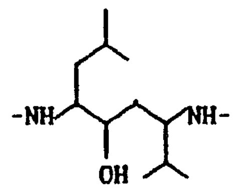 | Leu | - | 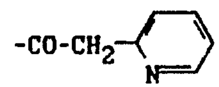 |
| 8 | Boc | - | - | 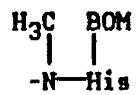 | 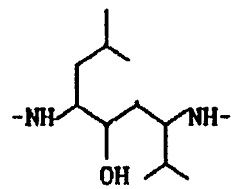 | Leu | - | 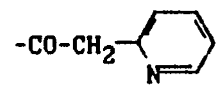 |
| 9 | Boc | - | Phe | His | 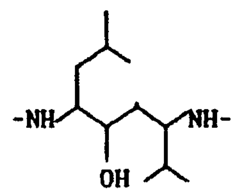 | - | - | 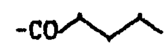 |
| 10 | Boc | Pro | Phe | 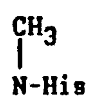 | 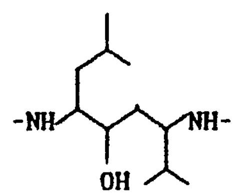 | Leu | - | 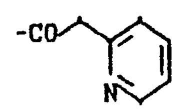 |

| Bsp.-Nr. | X | A | B | D | Y | E | F | Z |
|---|---|---|---|---|---|---|---|---|
| 11 | Boc | Pro | Phe | His | 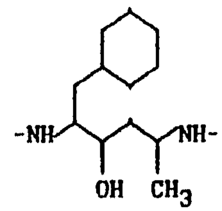 | Leu | - | 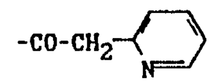 |
| 12 | Boc | - | Phe | His | 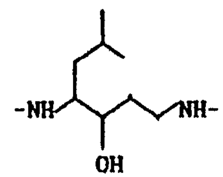 | - | - | Adamantyl |
| 13 | H | - | - | - | 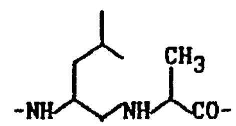 | Ile | - | 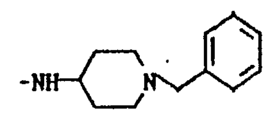 |
| 14 | Boc | - | Phe | His | 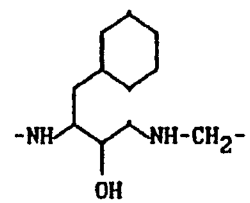 | - | - | 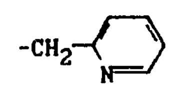 |
| 15 | Boc | - | Phe | His | 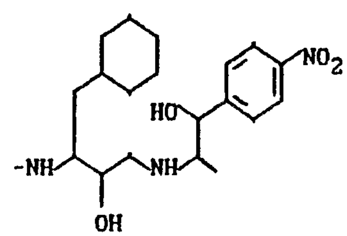 | - | - | $-CH_2-OH$ |

| Bsp.-Nr. | X | A | B | D | Y | E | F | Z |
|---|---|---|---|---|---|---|---|---|
| 16 | Boc | - | Phe | His | 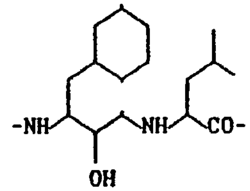 | - | - | $-NH_2$ |
| 17 | 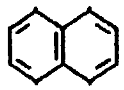 | - | $-N(Naph)-CH_2-CH_2-CO-$ | Phe | 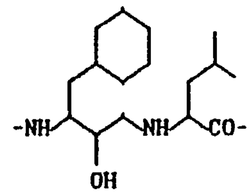 | - | - | $-NH_2$ |
| 18 | Boc | - | Phe | Ile | 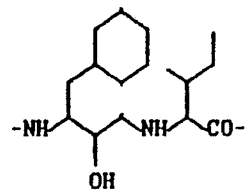 | - | - | 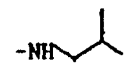 |
| 19 | Boc | - | Phe | His | 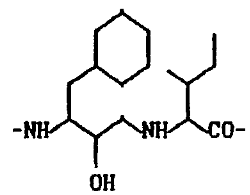 | - | - | 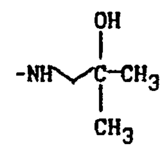 |
| 20 | Boc | - | Phe | His | 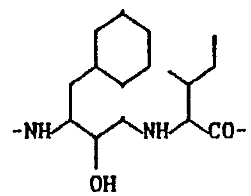 | - | - | 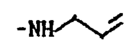 |

| Bsp.-Nr. | X | A | B | D | Y | E | F | Z |
|---|---|---|---|---|---|---|---|---|
| 21 | Boc | - | Phe | His | 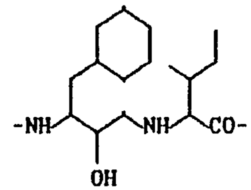 | - | - | 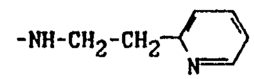 |
| 22 | Boc | - | Phe | His | 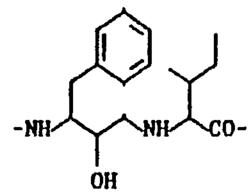 | - | - | 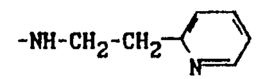 |
| 23 | Boc | - | - | - | 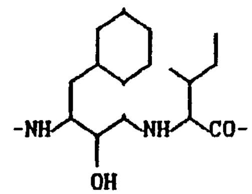 | - | - | 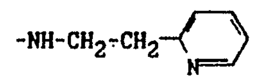 |
| 24 | Boc | - | - | His(Boc) | 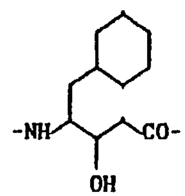 | Ile | - | 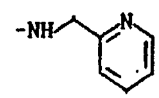 |
| 25 | H | - | - | - | 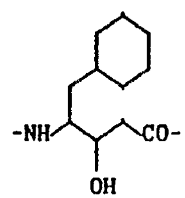 | Ile | | $-OCH_3$ |

Abkürzungen in der Tabelle:
BOC = tert.-Butyloxycarbonyl
Ile = Isoleucin
Phe = Phenylalanin
His = Histidin
Pro = Prolin
Leu = Leucin
Naph = Naphthyl

BOM = Benzyloxymethyl

Die erfindungsgemäßen Stoffe sowie Verfahren zu ihrer Herstellung sind bekannt [vgl. PCT WP 88102374; EP-A 201 81 071; EP-A 201 32 304 und EP-A 201 72 347].

Es wurde im Rahmen von Untersuchungen, die zu der vorliegenden Erfindung führten, überraschend gefunden, daß die erfindungsgemäß zu verwendenden Verbindungen der allgemeinen Formel (I) eine außerordentlich starke Wirkung gegen Retroviren besitzen. Dies wird durch die weiter unten angegebenen Versuchsdaten beispielhaft für die Verbindungen am Visna-Virus in Zellkulturen belegt. Das Visna-Virus und das HIV-Virus (Virus der humanen Immundefiziens) gehören beide zu der Retrovirus-Subfamilie der Lentiviren [Haase A.T. Nature (1986) 322, 130-136]. Beide Viren weisen eine ähnliche Genomorganisation und ein gegenüber den anderen Retroviren komplexes Transkriptionsmuster auf [Sonijo P. et al., Cell (1985), 42, 369 - 382; Davis J. L. et al., Journal of Virology (1987) 61 (5) 1325 - 1331].

Darüberhinaus wurde gezeigt [Frank, K.B., et al., Antimicrobial Agents and Chemotherapy (1987) 31 (9), S. 1369 - 1374], daß bekannte Inhibitoren des HIV auch das Visnavirus in vitro in vergleichbaren Konzentrationen hemmen; d.h, dieses Modell eignet sich für die Prüfung und Auffindung von Hemmstoffen des HIV.

In Zellkulturen, die mit Visna-Virus infiziert sind, treten 5 bis 10 Tage nach der Infektion ausgeprägte virus-induzierte, zytopathische Effekte auf. Durch Behandlung der infizierten Zellkulturen mit den erfindungsgemäßen Verbindungen konnte das Auftreten dieser zytopathischen Effekte verhindert werden.

Der Visna-Virus-Test wurde nach der Methode von O. Narayan et al., Journal of Infektious Diseases 135, 5, 1977, 800 - 806 durchgeführt. Dazu wurde die erfindungsgemäßen Verbindungen im Kulturmedium in nicht zytotoxischen Konzentrationen in 96er Microtiterplatten verdünnt. Anschließend wurden Fibroblastenzellen vom Schaf (5 x $10^4$ Zellen pro Napf) in Produktionsmedium zu jedem Näpfchen zugeführt. Jedes Näpfchen erhielt dann 50 $\mu$l einer Visna-Viruslösung mit einer Titer von ca. 2,5 x $10^4$ $TCID_{50}$ (TCID = tissue culture infectious dosis). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle ohne Substanz ein virusinduzierter, zytopathischer Effekt. Die infizierten und behandelten Zellen und die Kontrollzellen wurden 7 Tage bei 37 °C 5% $CO_2$ inkubiert.

Bei Auftreten des virusinduzierten zytopathogenen Effektes in der unbehandelten Viruskontrolle wurden die Kulturen mit Formalin fixiert und anschließend mit einer Giemsal-Lösung gefärbt. Die inhibitorische Konzentration ($IC_{50}$) wurde mikroskopisch als die Konzentration ermittelt, bei der der zytopathische Effekt um 50% gehemmt wurde: im Vergleich zur unbehandelten Viruskontrolle, die 100% Zellzerstörung aufwies.

Es wurde gefunden, daß beispielsweise die Verbindung aus Beispiel 20 die mit Visna-Virus infizierten Zellen vor der virusinduzierten Zellzerstörung schützt.

Tabelle 2

| Beispiel-Nr. | $IC_{50}$ (M) |
|---|---|
| 7 | 6.1 x $10^{-5}$ |
| 15 | 1.6 x $10^{-4}$ |
| 20 | 5 x $10^{-5}$ |

Die erfindungsgemäß zu verwendenden Verbindungen stellen somit wertvolle Wirkstoffe zu Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung oder Prophylaxe von menschlichen Retrovirusinfektionen.

2.) Für die Behandlung oder Prophylaxe von HIV II (Virus der humanen Immundefizienz; früher HTLV III/LAV genannt) verursachten Erkrankungen (AIDS) und den damit assozierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV I-oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxythylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-

und geschmacksverbesserte Zusätze, z.B. Pfefferminzöl und Eukalyptusöl und Süßmittel, z.B. Saccharin, enthalten.

Die Wirkstoffe der Formel (I) sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäß zu verwendenden Verbindungen können in den üblichen Konzentrationen und Zubereitungen zusammen mit dem Futter bzw. mit Futterzubereitungen oder mit dem Trinkwasser gegeben werden.

## Ansprüche

Peptide der allgemeinen Formel (I)

X-A-B-D-Y-E-F-Z (I)

in welcher

X - für Wasserstoff, geradkettiges oder verzweigtes Alkyl, Alkylsulfonyl oder Alkoxycarbonyl mit bis zu 10 Kohlenstoffatomen steht,

- für eine Gruppe der Formel $-COR^1$ steht,

worin

$R^1$ - geradkettiges oder verzweigtes Alkyl mit bis zu 8 Kohlenstoffatomen bedeutet, das gegebenenfalls durch Hydroxy, Amino oder Aryl mit 6 bis 10 Kohlenstoffatomen substituiert ist,

- für Phenylsulfonyl, Tolylsulfonyl oder Naphthyl steht, oder

- für eine Aminoschutzgruppe steht,

A - für eine direkte Bindung steht, oder

- für einen Rest der Formel

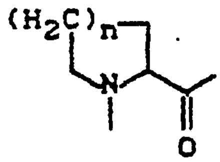

steht,

worin
n eine Zahl 1 oder 2 bedeutet,
- für eine Gruppe der Formel

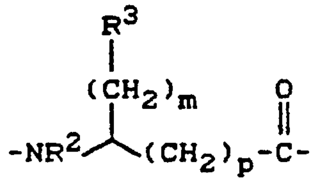

steht,

worin
m eine Zahl 0, 1, 2, 3 oder 4 bedeutet,
p eine Zahl 0 oder 1 bedeutet,
$R^2$ - Wasserstoff, geradkettiges oder verzweigtes Alkyl mit bis zu 8 Kohlenstoffatomen, Naphthyl oder eine Aminoschutzgruppe bedeutet,
$R^3$ - Wasserstoff, Naphthyl, Halogen, Carboxy, geradkettiges oder verzweigtes Alkyl oder Alkylthio mit bis zu 8 Kohlenstoffatomen bedeutet, das gegebenenfalls durch Mercapto, Alkylcarbonyl mit bis zu 8 Kohlenstoffatomen, Hydroxy oder Halogen substituiert ist, oder - eine Gruppe der Formel $-CH_2-NHR^4$ bedeutet,
worin
$R^4$ - Wasserstoff, geradkettiges oder verzweigtes Alkyl oder Alkylsulfonyl mit bis zu 8 Kohlenstoffatomen, Phenylsulfonyl oder eine Aminoschutzgruppe bedeutet,
- Guanidino, Indolyl, Imidazolyl, Pyridyl, Triazolyl oder Pyrazolyl bedeutet, die gegebenenfalls durch $R^4$ substituiert sind,
wobei
$R^4$ die oben angegebene Bedeutung hat,
- Aryl mit 6 bis 10 Kohlenstoffatomen bedeutet, das gegebenenfalls bis zu 3-fach gleich oder verschieden durch geradkettiges oder verzweigtes Alkyl oder Alkoxy mit bis zu 8 Kohlenstoffatomen, Halogen, Hydroxy, Nitro oder durch eine Gruppe der Formel $-NR^5R^6$ substituiert ist,
worin
$R^5$ und $R^6$ gleich oder verschieden sind und Wasserstoff oder geradkettiges oder verzweigtes Alkyl mit bis zu 8 Kohlenstoffatomen bedeuten,
B, E und F gleich oder verschieden sind und die oben angegebene Bedeutung von A haben und mit dieser gleich oder verschieden sind, in ihrer D-Form, L-Form oder D,L-Isomerengemisch, bevorzugt in der L-Form und
D - die oben angegebene Bedeutung von A hat und mit dieser gleich oder verschieden ist, in seiner D-Form, L-Form oder als D,L-Isomerengemisch, mit der Maßgabe, daß D nicht für eine direkte Bindung und nicht für Asparagin (Asn) stehen darf,
Y - für eine Gruppe der Formel

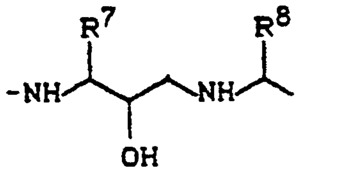 , 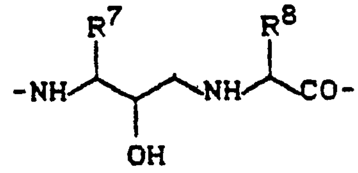

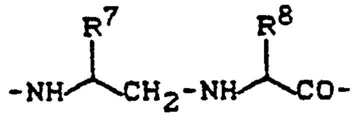 , 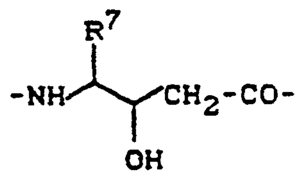

oder 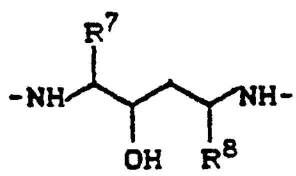 steht,

worin $R^7$ - geradkettiges oder verzweigtes Alkyl mit bis zu 12 Kohlenstoffatomen bedeutet, das gegebenenfalls durch Cycloalkyl mit 3 bis 8 Kohlenstoffatomen, Aryl mit 6 bis 10 Kohlenstoffatomen, Halogen, Hydroxy, Nitro oder durch einen gesättigten oder ungesättigten 5-bis 7-gliedrigen Heterocyclus mit bis zu 4 Heteroatomen aus der Reihe Sauerstoff, Schwefel oder Stickstoff substituiert ist,

- Aryl mit 6 bis 10 Kohlenstoffatomen bedeutet, das gegebenenfalls durch Halogen, Nitro, Hydroxy, Cyano, geradkettiges oder verzweigtes Alkyl oder Alkoxy mit bis zu 8 Kohlenstoffatomen oder durch eine Gruppe der Formel $-NR^5R^6$ substituiert ist,

worin

$R^5$ und $R^6$ die oben angegebene Bedeutung haben,

$R^8$ - Wasserstoff, geradkettiges oder verzweigtes Alkyl mit bis zu 8 Kohlenstoffatomen bedeutet, das gegebenenfalls durch Hydroxy, Cycloalkyl mit 3 bis 8 Kohlenstoffatomen und/oder Aryl mit 6 bis 10 Kohlenstoffatomen substituiert ist, welches seinerseits durch Nitro, Cyano oder Hydroxy substituiert ist,

Z - für eine Aminoschutzgruppe steht,

- für geradkettiges oder verzweigtes Alkyl oder Alkoxy mit bis zu 10 Kohlenstoffatomen steht, die gegebenenfalls durch Hydroxy, Phenyl oder Pyridyl substituiert sind,

- für eine Gruppe der Formel $-NR^9R^{10}$ oder $-COR^{11}$ steht,

worin

$R^9$ und $R^{10}$ gleich oder verschieden sind und Wasserstoff, geradkettiges oder verzweigtes Alkyl oder Alkenyl mit bis zu 10 Kohlenstoffatomen bedeuten, die gegebenenfalls durch Pyridyl oder Hydroxy substituiert sind, oder einen gesättigten 5-bis 7-gliedrigen Heterocyclus mit bis zu 3 Heteroatomen aus der Reihe Schwefel, Sauerstoff oder Stickstoff bedeuten, der gegebenenfalls durch Benzyl oder Pyridyl substituiert ist,

$R^{11}$ geradkettiges oder verzweigtes Alkyl mit bis zu 8 Kohlenstoffatomen bedeutet, das gegebenenfalls durch Pyridyl oder durch eine Gruppe der Formel $-NR^9R^{10}$ substituiert ist,

worin

$R^9$ und $R^{10}$ die oben angegebene Bedeutung haben,

- für Adamantyl oder für einen gesättigten oder ungesättigten 7-gliedrigen Heterocyclus mit bis zu 3 Heteroatomen aus der Reihe Sauerstoff, Schwefel oder Stickstoff steht, der gegebenenfalls durch Benzyl substituiert ist,

zur Behandlung von retroviralen Infektionen.